# EUROPÄISCHE PATENTANMELDUNG

(11) **EP 0 671 409 A2**
(43) Veröffentlichungstag der Anmeldung: **13.09.1995**
(21) Anmeldenummer: 95103161.6
(22) Anmeldetag: 06.03.1995
(51) Int. Cl.: C07H 15/207, A61K 31/70, C07H 17/04, G01N 33/569, G01N 33/574

(54) **Malonsäurederivate mit antiadhäsiven Eigenschaften**

(30) Priorität: 11.03.1994 DE 4408248; 25.08.1994 DE 4430005
(71) Anmelder: HOECHST AKTIENGESELLSCHAFT, D-65929 Frankfurt am Main (DE)
(72) Erfinder: Toepfer, Alexander, Dr., D-65719 Hofheim (DE); Kretzschmar, Gerhard, Dr., D-65760 Eschborn (DE); Bartnik, Eckart, Dr., D-65205 Wiesbaden (DE); Seiffge, Dirk, Dr., D-55246 Mainz-Kostheim (DE)

(57) **Zusammenfassung**

Die Erfindung betrifft Malonsäurederivate mit antiadhäsiven Eigenschaften der Formel I 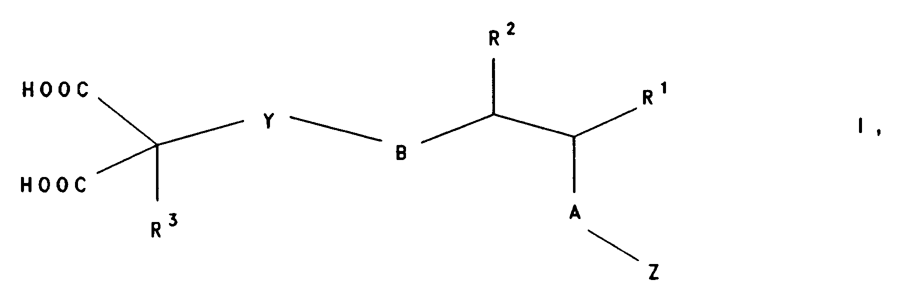 in der
R¹, R² und R³ unabhängig voneinander H, (CH₂)ₘX oder CH₂O(CH₂)ₘX¹ bedeuten oder
R¹ und R² gemeinsam einen sechsgliedrigen Carbo- oder Heterocyclus mit mindestens einem der Substituenten R⁴, R⁵ und R⁶ bilden und
A und B unabhängig voneinander O, S, NH, HN-CO, OC-NH, O-CO, OC-O, NH-CO-O, O-CO-NH, S-CO, SC-O, O-CS-S, S-CS-O, NH-CS-S, S-CS-NH oder CH₂ bedeuten und
Z eine Pyranose, eine Furanose, einen offenkettigen Polyalkohol darstellt oder Y-X⁶ bedeutet,
Y -O-(CX²,X³)ₙ, -(CX²,X³)ₙ, -(CX²,R⁷)ₙ-, -(CR⁷,R⁸)ₙ-, - CH₂-(CX²,X³)-ₙ oder einen gesättigten oder ungesättigten, sechsgliedrigen Carbo- oder Heterozyklus mit mindestens einem Substituenten R⁹ oder eine Kombination aus der Kette -O-(CX²,X³)ₙ,-(CX²,X³)ₙ, -(CX²,R⁷)ₙ-, -(CR⁷,R⁸)ₙ- und dem Carbo- oder Heterozyklus darstellt,
   wobei
R⁴, R⁵ und R⁶ unabhängig voneinander H, OH, (CH₂)qX⁴,CH₂0(CH₂)qX^{S} oder HNC(O)CH₃ und
R⁷, R⁸ und R⁹ unabhängig voneinander H, OH, (CH₂)qX⁴ oder CH₂0(CH₂)qX⁵ und
X, X¹, X², X³, X⁴ und X⁵ unabhängig voneinander H, NH₂, COOH, OH, CH₂0H, CH₂NH₂, C₁-C₂₀-Alkyl oder C₆-C₁₀-Aryl und
_{X}6 OH oder 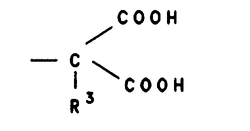 und
m, n und q unabhängig voneinander eine Zahl von 1 bis 20 bedeuten,

ein Verfahren zu deren Herstellung, deren Verwendung sowie aus diesen hergestellte Arzneimittel und Diagnostika.

## Beschreibung

Die Erfindung betrifft Malonsäurederivate mit antiadhäsiven Eigenschaften, ein Verfahren zu deren Herstellung, deren Verwendung sowie aus diesen hergestellte Arzneimittel und Diagnostika.

Die auf der Oberfläche eukaryontischer Zellen befindlichen Glycoconjugate stellen spezifische Bindungsepitope für Transmembran-Proteine dar, welche man als Selektine bezeichnet. Diese Selektine, welche sowohl im Endothel als auch auf den verschiedenen zirkulierenden Zellen des hämatolymphoiden Systems vorkommen, gehen spezifische Wechselwirkungen mit Kohlenhydrat-Epitopen (Liganden) des jeweils anderen Zelltyps ein (K.-A. Karlsson, TIPS 12, (1991), 265-272).

Synthetische Analoga der Selektin-Liganden sind daher vielversprechende Kandidaten für Antiphlogistika und Antikoagulantien (T.A. Springer, L.A. Lasky, Nature 349, 1991, 196-197; T. Feizi, TIBS 16, 1991, 84-86). Auch als Erkennungsdomänen für Viren (J.C. Paulson, The Receptors, Vol. 11, Ed.: P. M. Conn, Academic press, 1985, 131), Bakterien (Strömberg et al. EMBO J. 1990, (9), 2001) und Toxine (Karlsson et al. Sourcebook of Bacterial Protein Toxins, Eds. J.E. Alouf, J.H. Freer, Academic Press, 1990, (56), 3537) spielen Kohlenhydratliganden eine entscheidende Rolle und damit auch bei der Einleitung der entsprechenden Krankheiten (Prophylaxe, Diagnostik oder Therapie von bakteriellen und viralen Infektionen, entzündlichen Erkrankungen, rheumatische Arthritis, Allergien, Nachinfarktsyndrom, Schock, Schlaganfall, akute und chronische Organabstoßung, Vasculitis, Sepsis, Schocklunge, usw.).

Da Krebszellen ein anderes Muster dieser Kohlenhydratstrukturen aufweisen als gesunde Zellen, ermöglicht dies die Verwendung dieser Mimetika einerseits als Marker, andererseits zur Bekämpfung von Tumorzellen, insbesondere bei metastastatisierenden Tumoren (S.-i. Hakomori, Cancer Cells, December 1991, Vol. 3, No. 12).

Von besonderer Bedeutung bei der Einleitung der oben genannten Erkrankungen sind silylierte und/oder fucosylierte Kohlenhydrate (Sialic Acids in "Cell Biology Monographs" Schauer, Editor, Vol. 10, 1982). Besonders als Mediatoren der Zelladhäsion spielen diese eine entscheidende Rolle (Lowe et al., Cell, 63, 475-485, 1990). Eine besondere Bedeutung kommt in diesem Zusammenhang Sialyl-Lewis-X [aNeu5Ac(2-+3)ßGal(1-+4)[aFuc(1-+3)]-ßGlcNAc-OR] und Sialyl-Lewis-A [aNeu5Ac(2-3)ßGal(1-3)[aFuc-(1→4]-βGIcNAc-OR] zu (R ist definiert als ein Aglycon mit mindestens einem Kohlenstoffatom). Für diese Verbindungen wurden sowohl chemische (Ratcliffe et al., U.S. Patent No. 5,079,353) als auch chemisch/enzymatische Synthesen entwickelt (A. Venot et al., PCT/CA 92/00251).

Mit dem Ziel einer Vereinfachung der sehr aufwendigen Synthesen dieser Verbindungen bei Erhaltung oder Verbesserung ihrer Bindungsaffinitäten zu den entsprechenden Selektinen wurden bereits mehrere Strukturen vorgeschlagen und teilweise synthetisiert. So wurde beispielsweise Neuraminsäure durch Milch-oder Glycolsäure und/oder Fucose durch Glycerin bzw. Trifluormethyl-Fucose und/oder N-Acetylglucosamin durch Glucosamin oder Glucose ersetzt (PCT/US 92/09870). Eine Substitution von Neuraminsäure durch Sulfat oder Phosphat ist ebenfalls beschrieben (PCT/CA 92/00245). Beschrieben ist außerdem eine Substitution von Glucosamin durch eine Kette von mindestens 2 Kohlenstoffatomen (WO 92/18610). Nach dem heutigen Stand der Technik ist lediglich für das native Sialyl-Lewis-X und Sialyl-Lewis-A mit geringfügigen Modifikationen eine effiziente, enzymatische "Large Scale-Synthese" entwickelt worden (C.H. Wong et al., WO 92/16640 und US 5,162,513). Diese Verbindungen haben jedoch den Nachteil einer geringen Affinität zu den Selektinen zum einen und einer geringen in vivo-Stabilität zum anderen (Wirkstoffe sind nicht oral verfügbar).

Demgegenüber ist es Aufgabe der vorliegenden Erfindung, neue, physiologisch stabilere Selektin-Liganden bereitzustellen, welche eine gegenüber den natürlichen Liganden vergleichbare oder stärkere Wechselwirkung mit dem Rezeptor aufweisen und sich im Vergleich zu den natürlichen Liganden leicht herstellen lassen.

Es ist ferner Aufgabe der vorliegenden Erfindung, auf Basis dieser neuen Selektin-Liganden Arzneimittel zur Therapie oder Prophylaxe und Mittel zur Diagnose von Krankheiten verfügbar zu machen, bei welchen bakterielle oder virale Infektionen, metastasierende Tumore oder entzündliche Prozesse in volviert sind.

Die gestellte Aufgabe wird gelöst durch

1. eine Verbindung der Formel I 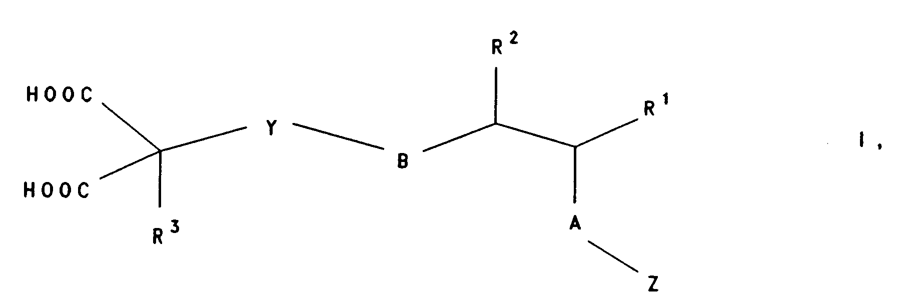 in der
   R¹, R² und R³ unabhängig voneinander H, (CH₂)ₘX oder CH₂O(CH₂)ₘX¹ bedeuten oder R¹ und R² gemeinsam einen sechsgliedrigen Carbo- oder Heterocyclus mit mindestens einem der Substituenten R⁴, R⁵ und R⁶ bilden und
   A und B unabhängig voneinander O, S, NH, HN-CO, OC-NH, O-CO, OC-O, NH-CO-O, O-CO-NH, S-CO, SC-O, O-CS-S, S-CS-O, NH-CS-S, S-CS-NH oder CH₂ bedeuten und
   Z eine Pyranose, eine Furanose, einen offenkettigen Polyalkohol darstellt oder Y-X⁶ bedeutet,
   Y -O-(CX²,X³)ₙ, -(CX²,X³)ₙ, -(CX²,R⁷)ₙ-, -(CR⁷,R⁸)ₙ-, -CH₂-(CX²,X³)-ₙ oder einen gesättigten oder ungesättigten, sechsgliedrigen Carbo- oder Heterozyklus mit mindestens einem Substituenten R⁹ oder eine Kombination aus der Kette -0-(CX²,X³)ₙ,-(CX²,X³)ₙ, -(CX²,R⁷)ₙ-, -(CR⁷,R⁸)ₙ- und dem Carbo-oder Heterozyklus darstellt, wobei
   R⁴, R⁵ und R⁶ unabhängig voneinander H, OH, (CH₂)_{q}X⁴,CH₂O(CH₂)_{q}X⁵ oder HNC(O)-CH₃ und
   R⁷, R⁸ und R⁹ unabhängig voneinander H, OH, (CH₂)qX⁴ oder CH₂O(CH₂)_{q}X⁵ und
   X, X¹, X², X³, X⁴ und X⁵ unabhängig voneinander H, NH₂, COOH, OH, CH₂0H, CH₂NH₂, C₁-C₂₀-Alkyl oder C₆-C₁₀-Aryl und
   _{X}6 OH oder 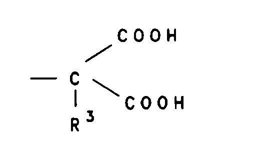
      und
   m, n und q unabhängig voneinander eine Zahl von 1 bis 20 bedeuten,
2. insbesodere durch eine Verbindung der Formel 1, die sich dadurch auszeichnet, daß
   R¹ und R² einen sechsgliedrigen Carbocyclus bilden und
   R³, R⁴, R⁵ und R⁶ H,
   A O,
   Z eine -Fucopyranosyl-Gruppe,
   Y (CX²,X³)ₙ und
   X² und X³ H bedeuten,
3. insbesondere durch eine Verbindung der Formel 1 gemäß Nr. 2, welche dadurch gekennzeichnet ist, daß B O bedeutet,
4. vorzugsweise dadurch gekennzeichnet, daß n für 3 steht, nämlich

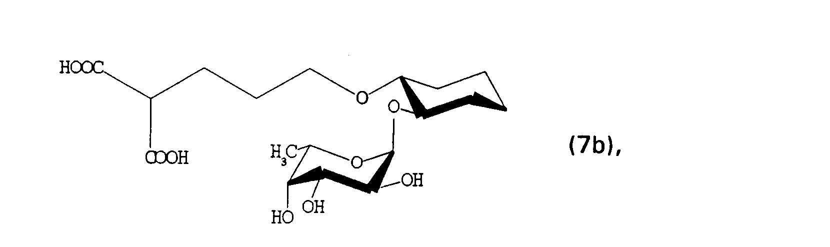

5. vorzugsweise dadurch gekennzeichnet, daß n für 4 steht, nämlich,

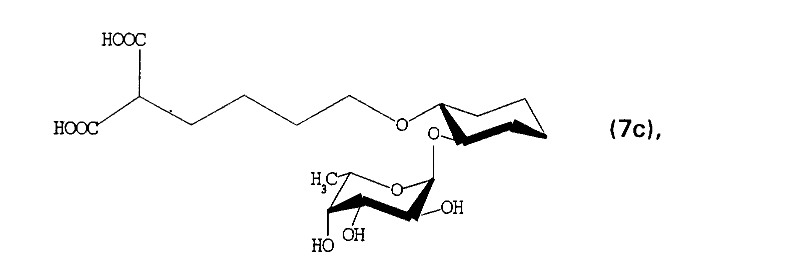

6. oder dadurch gekennzeichnet, daß n für 5 steht, nämlich 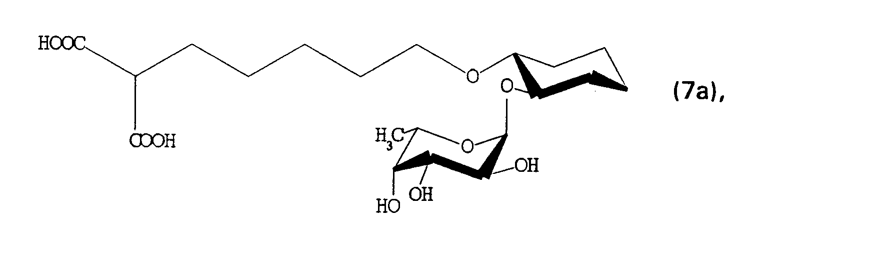
7. oder durch eine Verbindung gemäß Nr. 2, welche sich dadurch auszeichnet, daß B HNCO-O bedeutet,
8. vorzugsweise ferner dadurch gekennzeichnet, daß n für 4 steht, nämlich

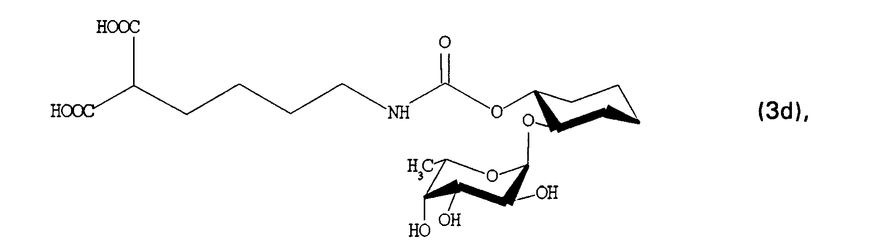

9. oder durch eine Verbindung gemäß Formel I, welche sich dadurch auszeichnet, daß
   R¹ und R² einen sechsgliedrigen Carbocyclus bilden und
   R³, R⁴, R⁵ und R⁶ H,
   A und B O,
   Z eine Fucopyranosyl-Gruppe,
   Y eine Kombination aus einer Kette -O-(CX²,X³)ₙ und einem Heterozyklus darstellt, wobei
   X² und X³ H bedeuten,
10. vorzugsweise dadurch gekennzeichnet, daß der Heterocyclus ein Galactosylrest ist und n für 2 steht, nämlich

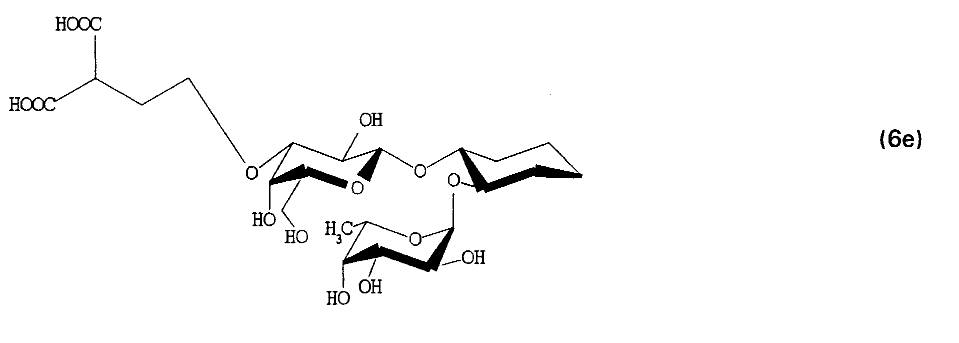

11. oder durch eine Verbindung der Formel I, welche sich dadurch auszeichnet, daß R¹ und R² gemeinsam einen substituierten Tetrahydropyranring bilden,
   A und B O,
   Z eine -Fucopyranosyl-Gruppe,
   Y (CX²,X³)ₙ bedeuten, wobei
   R³, X² und X³ H bedeuten und n für 4 steht,
12. insbesondere dadurch gekennzeichnet, daß die Substituenten des Tetrahydropyranrings
   R⁴ und R⁵ H und

   der im Tetrahydropyranring zwischen dem Ringheteroatom O und dem Ringsubstituenten -A-Z liegende Substituent
   R⁶ CH₂O(CH₂)_{q}X⁵ und
   X⁵ C₆ H₅ bedeuten,
13.vorzugsweise dadurch gekennzeichnet, daß q für 4 steht, nämlich 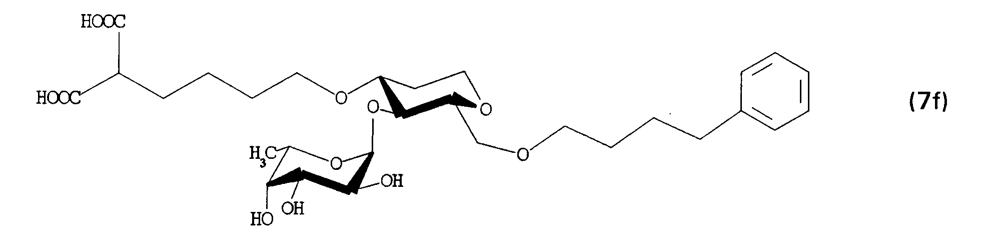
14. oder durch eine Verbindung der Formel I gemäß Nr. 11, welche sich dadurch auszeichnet, daß der Tetrahydropyranring eine Pyranose,
15. vorzugsweise N-Acetyl-D-glucosamin ist,
16.oder durch eine Verbindung der Formel I, welche dadurch gekennzeichnet ist, daß
   R¹ und R² gemeinsam einen sechsgliedrigen Carbocyclus bilden, A-Z

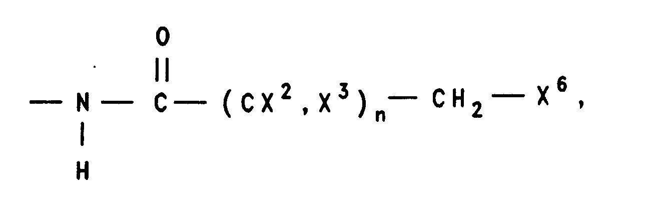

   -B-Y- 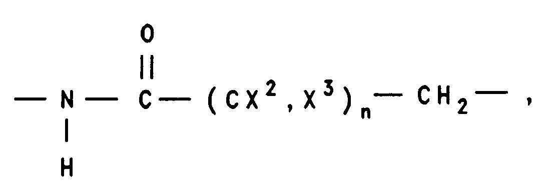
   R³ und X² H,
   X³ und X⁶ OH und
   n 4 bedeuten,
17. oder vorzugsweise
   X⁶ 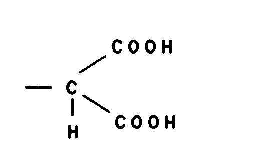 bedeutet.
18. Die eingangs gestellte Aufgabe wird ferner gelöst durch ein Verfahren zur Herstellung einer Verbindung der Formel I, welches dadurch gekennzeichnet ist, daß man zunächst unter Alkylierung, Acylierung oder Glycosylierung einer funktionellen Gruppe eines Akzeptors II, 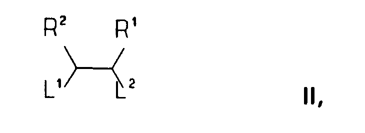 aufweisend mindestens zwei benachbarte funktionelle Gruppen L¹ und L² sowie aufweisend die Substituenten R¹ und R², mittels eines Äquivalentes eines mindestens zwei funktionelle Gruppen L³ und L⁴ tragenden Donors 111, 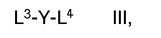 dessen eine funktionelle Gruppe L³ notwendigenfalls geschützt und dessen andere funktionelle Gruppe L⁴ gebenenfalls aktiviert vorliegt, Zwischenverbindung IV, 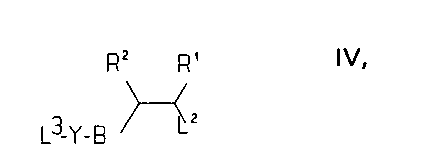 herstellt, wonach man unter Alkylierung, Acylierung oder Glycosylierung der ungeschützten funktionellen Gruppe L² der Zwischenverbindung IV mittels eines mit einer aktivierten funktionellen Gruppe L⁵ versehenen Donors V, 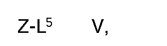 dessen übrige funktionelle Gruppen notwendigenfalls Schutzgruppen tragen, Zwischenverbindung VI, 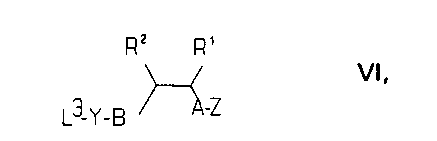 herstellt und schließlich gegebenenfalls nach selektiver Entschützung und Aktivierung der funktionellen Gruppe L³ durch Umsetzung der Zwischenverbindung VI mit einem Malonsäurederivat und nachfolgender Abspaltung sämtlicher Schutzgruppen die Verbindung der Formel I gemäß Nr. 1 erhält, wobei die Variablen R¹, R², Y, B, Z und A die in Anspruch 1 genannte Bedeutung haben.

19. Wahlweise kann man Akzeptor II zunächst mit Donor V und anschließend mit Donor III zur Zwischenverbindung VI umsetzen.
20. Vorzugsweise werden die Verbindungen gemäß Nr. 3, beispielsweise die Verbindungen 7a, 7b und 7c, dadurch hergestellt, daß man als Akzeptor II (1R,2R)-trans-1,2-Cyclohexandiol, als Donor III ein n-Allyloxy-1-p-toluolsulfonyloxy-(C₂-Cₙ)-alkan und als Donor V Thioethyl-O-2,3,4-tri-O-benzyl-ß-L-fucopyra- nosid einsetzt.
21. Eine Verbindung gemäß Nr. 7, beispielsweise Verbindung 3d, läßt sich unter Anwendung des unter Nr. 19 angegebenen erfindungsgemäßen Verfahrens vorzugsweise dadurch herstellen, daß man als Akzeptor II (1R,2R)-trans-1,2-Cyclohexandiol, als Donor III ein mit Chlorameisensäure-p-nitrophenylester aktivertes Aminoalkanol und als Donor V Thioethyl-O-2,3,4-tri-O-benzyl-ß-L-fucopyranosid verwendet.
22. Die unter Nr. 16 beschriebene Verbindung läßt sich unter Verwendung des erfindungsgemäßen Verfahrens vorzugsweise dadurch herstellen, daß man als Akzeptor II (1 R,2R)-trans-1,2-CycIohexandi- amin und als Donor III und V Glucono-1 ,5-lacton verwendet.
23. Die unter Nr. 17 beschriebene Verbindung läßt sich unter Verwendung des erfindungsgemäßen Verfahrens vorzugsweise dadurch herstellen, daß man als Akzeptor II (1 R,2R)-trans-1,2-CycIohexandi- amin, als Donor III und V Glucono-1,5-lacton verwendet und daß man nach selektiver Entschützung und Aktivierung der endständigen funktionellen Gruppen jeweils an Y und an Z Zwischen-verbindung VI mit zwei Äquivalenten eines entsprechenden Malonsäurederivates umsetzt.
24. Die gestellte Aufgabe wird ferner gelöst durch ein Arzneimittel, enthaltend eine der unter Nr. 1 bis 17 offenbarten Verbindungen der allgemeinen Formel I und gegebenfalls pharmazeutische Träger.
25. Die unter Nr. 1 bis 17 offenbarten Verbindungen der allgemeinen Formel I können vorteilhaft zur Herstellung von Arzneimitteln zur Therapie oder Prophylaxe von Krankheiten, bei welchen bakterielle oder virale Infektionen, entzündliche Prozesse oder metastasierende Tumoren involviert sind verwendet werden.
26. Die unter Nr. 1 bis 17 offenbarten Verbindungen der allgemeinen Formel I können vorteilhaft auch zur Herstellung eines Mittels zur Diagnose von Krankheiten verwendet werden, bei welchen bakterielle oder virale Infektionen, entzündliche Prozesse oder metastasierende Tumoren involviert sind.

Die erfindungsgemäßen Malonsäurederivate sind im Vergleich zu den natürlichen Kohlenhydratliganden und deren bisher bekannten Derivaten einfach und billig herzustellen und weisen zudem maximal zwei, in der Regel jedoch nur eine glycosidische Bindung auf, was wiederum eine erhöhte physiologische Stabilität der Produkte zur Folge hat.

Die Verbindungen dieser Erfindung mit der allgemeinen Formel I lassen sich ausgehend von käuflichen Komponenten mit mindestens 2 benachbarten funktionellen Gruppen wie z. B. (1 R,2R)-trans-1,2-Cyclohexandiol (Fluka), trans-2-Aminohexanol (Enantiomerentrennung: Takada H. et al, Bull. Chem. Soc. Jpn. (1994) 67, 4, 1196-97) oder trans-1,2-Cyclohexandiamin herstellen. Aus diesen Verbindungen können beispielsweise (im ersten Fall) durch Umsetzung mit höchstens einem Äquivalent des Tosylats (oder anders aktivierten Restes: Triflat, Mesylat, Halogenide usw.) eines beispielsweise monoallylierten Diols die entsprechend monoalkylierten Verbindungen synthetisiert werden.

Alternativ kann statt der Etherbindung auch eine andere Anknüpfung (Amid, Ester, Amin, Thioether,Thioester, Urethan, Xanthogenat oder Dithiocarbaminat) gewählt werden.

Die zweite funktionelle Gruppe wird nun mit einem aktivierten Monosaccharidbaustein (z.B. Thioethyl-O-2,3,4-tri-O-benzyl-ß-L-fucopyranosid oder 0-{2,3,4,6-Tetra-O-benzyl-a/ß-D-mannopyranosyll-trichloracetimi- dat) glycosyliert. Statt einem Monosaccharid kann auch ein Polyalkohol wie L-Threitol angeknüpft werden (über 1,2,3-Tri-O-benzyl-4-O-p-toluolsufonat). Auf diese Weise erhält man eine Verbindung ohne eine glycosidische Bindung. Nach Abspaltung der Allylschutzgruppe (oder einer anderen geeigneten Schutzgruppe) des zuerst angeknüpften monoallylierten Tosylats wird die freigewordene Hydroxylgruppe beispielsweise tosyliert und anschließend mit Malonsäuredimethylester (oder Malonsäuredibenzylester) alkyliert. Hydrogenolyse (und Verseifung) liefert das gewünschte Malonsäurederivat.

Alternativ kann ausgehend von (1 R,2R)-trans-1,2-Cyclohexandiamin durch Umsetzung mit 2 Äquivalenten Glucono-1,5-lacton eine Zwischenverbindung erhalten werden, die zwei identische Seitenketten mit jeweils einer primären und mit jeweils mehreren sekundären OH-Gruppen trägt und welche nach selektiver Schützung und Aktivierung nach bekannten Methoden, wie etwa die Aufeinanderfolge von Tritylierung, Benzylierung, Detritylierung und Tosylierung, sich beispielsweise mit Malonsäuredimethylester zum gewünschten Malonsäurederivat umsetzen läßt. Wahlweise kann durch entsprechende Variation der Schutzgruppentechnik sowie entsprechende Aktivierung der primären OH-Gruppen beider Seitenketten eine Verbindung erhalten werden, die zwei Malonsäuregruppen - bzw. jeweils endständig an jeder der beiden Seitenketten eine Malonsäuregruppe - trägt.

Durchführung von HL60 Zelladhäsionsassays auf rekombinanten, löslichen Adhäsionsmolekülen

Die erfindungsgemäßen Malonsäurederivate können trotz ihrer wesentlich geringeren Molmasse eine höhere Affinität zu den natürlichen Rezeptoren, beispielsweise zu E- und P-Selektin, als die natürlichen Kohlenhydratliganden haben, wie anhand der nachfolgend beschriebenen Zelladhäsionsassays nachgewiesen werden kann.

1. 96er Mikrotitertestplatten (Nunc Maxisorb) werden mit 100 µl eines in 50 mM Tris pH 9,5 verdünnten (1 + 100) Ziege anti human IgG Antikörpers (Sigma) 2 Std. bei Raumtemperatur inkubiert. Nach Entfernen der Antikörperlösung wird einmal mit PBS gewaschen.
2. 150 µl des Blockierungspuffers werden für 1 Std. bei Raumtemperatur in den Näpfchen belassen. Die Zusammensetzung des Blockierungspuffers ist:

0,1 % Gelatine, 1 % BSA, 5 % Kalbserum, 0,2 mM PMSF, 0,02 % Natriumazid. Nach Entfernen des Blockierungspuffers wird einmal mit PBS gewaschen.

3. In die Näpfchen werden je 100 µl Zellkulturüberstand von entsprechend transfektierten und exprimierenden COS-Zellen pipettiert. Die Inkubation erfolgt 2 Std. bei Raumtemperatur. Nach Entfernen des Zellkulturüberstandes wird einmal mit PBS gewaschen.
4. In die Näpfchen werden 20 µl Bindunspuffer gegeben. Der Bindungspuffer hat die Zusammensetzung: 50 mM Hepes, pH 7,5; 100 mM NaCI; 1 mg/ml BSA;

2 mM MgCI₂; 1mM CaC1₂; 3 mM MnC1₂; 0,02 % Natriumazid; 0,2 mM PMSF. Dazu werden 5 µl der Testsubstanz pipettiert, durch Schwenken der Platte vermischt und 10 Min. bei Raumtempeartur inkubiert.

5. 50 ml einer HL60 Zellkultur mit 200.000 Zellen/ml werden 4 Min. bei 350 g zentrifugiert. Das Pellet wird in 10 ml RPMI 1640 resuspendiert und die Zellen erneut zentrifugiert. Zur Markierung der Zellen werden 50 ug BCECF-AM (Molecular Probes) in 5 µl wasserfreiem DMSO aufgelöst; anschließend werden 1,5 ml RPMI 1640 auf die BCECF-AM/DMSO-Lösung gegeben. Mit dieser Lösung werden die Zellen resuspendiert und 30 Min. bei 37°C inkubiert. Nach zweiminütiger Zentrifugation bei 350 g wird das markierte Zellpellet in 11 ml Bindungspuffer resuspendiert und die resuspendierten Zellen in 100 µl Aliquots in die Mikrotiterplatten-Näpfchen verteilt. Die Platte wird 10 Min. bei Raumtemperatur stehen gelassen, um die Zellen auf den Boden der Testplatte sedimentieren zu lassen. Dabei haben die Zellen Gelegenheit an das beschichtete Plastik zu adhärieren.

6. Zum Abstoppen des Tests wird die Mikrotiterplatte im 45 _{°} Winkel gänzlich in den Stoppuffer getaucht (25 mM Tris, pH 7,5; 125 mM NaCI; 0,1 % BSA; 2 mM MgCI₂; 1 mM CaC1₂; 3 mM MnC1₂; 0,02 % Natriumazid). Durch Invertierung wird der Stoppuffer aus den Näpfchen entfernt und die Prozedur noch zweimal wiederholt.

7. Die Messung der in den Näpfchen festhaftenden, BCECF-AM-markierten Zellen erfolgt in einem Cytofluorimeter (Millipore), bei einer Empfindlichkeitseinstellung von 4, einer Anregungswellenlänge von 485/22 nm und einer Emissionswellenlänge von 530/25 nm.

### Ergebnisse

IC 50-Werte für E-Selektin [mM], in runden Klammern für P-Selektin [mM]:
5-Malonyl-1-hydroxypentyl-(1→1)-[α-L-fucopyranosyl)-(1→2)]-(1R,2R)-trans-1,2-cyclohexandiol (7a): 0,7-1,1 (0,32-0,43)

(Die Werte für Sialyl-Lewis X liegen für E-Selektin etwas, für P-Selektin sogar deutlich höher (jeweils 2 mmol) und sind damit schlechter.)
4-Malonyl-1-hydroxybutyl-(1→1)-[a-L-fucopyranosyl)-(1→2)]-(1R,2R)-trans-1,2-cyclohexandiol (7c):
nicht meßbar (0,175)
4-Malonyl-1-aminocarbonylbutyl-(4→2)-[(α-L-fucopyranosyl)-(1→1)]-(1 R,2R)-trans-1,2-cyclohexandiol (3d):
   größer als 1 (0,0065)
   (1 R,2R)-trans-1,2-Cyclohexandiol-O-(a-L-fucopyranosyl)-ß-D-2-O-malonylethyl-galactopyranosid (6e):
   1,4 (0,018)
   4-Malonyl-1-hydroxybutyl-(1→3)-[(α-L-fucopyranosyl)-(1→4)]-[4-phenyl-1-hydroxybutyl-(1→6)]-1,2- didesoxyglucose (7f):
   0,34 (0,017)

Die Malonsäurederivate gemäß der vorliegenden Erfindung stellen allesamt potentielle Liganden für die bisher bekannten Selektine (Proteine, welche auf dem Endothel und anderen Zelloberflächen exprimiert werden und an spezifische Kohlenhydrat-Liganden binden) dar.

Die erfindungsgemäßen Malonsäurederivate können demzufolge als Anti-Adhäsionstherapeutika eingesetzt werden und verhindern im Fall von Entzündungen, daß die ELAM-1-Rezeptoren auf stimulierten Endothelzellen an Sialyl-Lewis-X-Strukturen auf der Oberfläche von Leukozyten binden. Im Fall der Influenza-Therapie verhindern die Malonsäurederivate die Anheftung von Viren an die Neuraminsäure auf der Zelloberfläche und damit auch die Endozytose der Viruspartikel.

Aus diesem Sachverhalt ergibt sich die Möglichkeit zur Prophylaxe, Diagnostik oder Therapie Selektinvermittelter Krankheiten. Hierzu gehören beispielsweise:
1. Autoimmunkrankheiten:
   Rheumatische Arthritis, Multiple Sklerose, entzündliche Knochenerkrankungen, Lupus, Myasthenia gravis, Allergien, Osteoarthritis, Asthma, Kontakt-Dermatitis, Psoriasis, Adult respiratory distress syndrome, Transplantatabstoßung.

2. Infektionen:
   Schnupfen, Grippe, Helicobacter pylori, Malaria, Septischer Schock.

3. Krebs:
   Colorectal, Brust, Eierstöcke, Prostata.
4. Zentrales Nervensystem:
   Schlaganfall, Trauma
5. Reperfusionsverletzungen:
   Myocardinfarkt, Angioplastie, instabile Angina, systemischer Schock
6. Weitere: Osteoporose, Wunden und schwere Verbrennungen.

Die erfindungsgemäßen Arzneimittel werden im allgemeinen intravenös, oral oder parenteral oder als Implantate verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro- )Kapseln, Zäpfchen, Sirupe, Emulsionen, Suspensionen, Aerosole, Tropfen oder injizierbare Lösungen in Ampullenform sowie Präparate mit protrahierte Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z. B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, Alkohole, Glycerin und mehrwertige Alkohole genannt.

Vorzugsweise werden die pharmazeutischen Präparate in Dosierungseinheiten hergestellt und verabreicht. Feste Dosierungseinheiten sind Tabletten, Kapseln und Suppositorien.

Für die Behandlung eines Patienten sind je nach Wirksamkeit der Verbindung, Art der Applikation, Art und Schwere der Erkrankung, Alter und Körpergewicht des Patienten, unterschiedliche Tagesdosen notwendig. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleiner Dosierungseinheiten als auch durch Mehrfachgabe unterteilten Dosen in bestimmten Intervallen erfolgen. Die zu verabreichende Tagesdosis kann außerdem von der Anzahl der während des Krankheitsverlaufs exprimierten Rezeptoren abhängig sein. Es ist vorstellbar, daß im Anfangsstadium der Krankheit nur wenige Rezeptoren auf der Zelloberfläche expirmiert werden und demzufolge die zu verabreichende Tagesdosis geringer ist als bei stark erkrankten Patienten.

Die erfindungsgemäßen Arzneimittel werden dadurch hergestellt, daß man ein Malonsäurederivat gemäß der vorliegenden Erfindung mit üblichen Träger- sowie gegebenenfalls Zusatz- und/oder Hilfsstoffen in die bzw. eine geeignete Darreichungsform bringt.

Die Verbindungen gemäß der vorliegenden Erfindung sind auch zur Herstellung von Antikörpern zur diagnostischen Bestimmung von Liganden geeignet, welche nicht zugänglich, nicht immunogen genug oder unbekannt sind.

Bei vielen Autoimmunerkrankungen und Tumoren sind bestimmte Liganden bzw. Antigene auf der Zellmembran hochreguliert. Diese sind aber häufig nicht bekannt, lassen sich nicht rein isolieren oder sind nicht genug immunogen, um daraus Antikörper herstellen zu können.

Die Verbindungen gemäß der vorliegenden Erfindung können zur Gewinnung von Antikörpern dienen, welche mit Epitopen der natürlichen, unbekannten bzw. nicht zugänglichen Liganden kreuzreagieren. Neben den diagnostischen Anwendungen sind für die auf diese Weise gewonnenen Antikörper auch therapeutische Anwendungen denkbar (A. N. Houghton, D. A. Scheinberg, Semin. Oncol. 13 (1986) 165-179; W. C. Eckelmann, In Vivo Diagnosis and Therapy of Human Tumors with Monoclonal Antibodies; Pergamon Press,London 1988; M. H. Ravindranath, D. L. Morton, R. F. Irie, Cancer Res. 49 (1989) 3891-3897).

### Spezielle Ausführungsbeispiele

### Beispiel 1

a) Synthese von 5-Allyloxy-1-p-toluolsulfonyloxy-pentan (1a): Eine Mischung aus Pentandiol (21.8 ml, 207 mmol), Allylbromid (11.7 m, 138 mmol), Kaliumcarbonat (21 g, 151.8 mmol) und Dibenzo-18-crown-6 wird 24 h im Ultraschallbad behandelt. Anschließend wird mit Dichlormethan (250 ml) verdünnt und 2 x mit Wasser (je 100 ml) gewaschen. Die organische Phase wird über Magnesiumsulfat getrocknet, eingeengt und der Rückstand mit Pyridin (50 ml, 600 mmol), Dichlormethan (700 ml) und p-Toluolsulfonsäurechlorid (40 g, 207 mmol) gerührt. Nach 16 h wird mit gesättigter Kochsalzlösung gewaschen, die organische Phase getrocknet und eingeengt. Flash-Chromatographie (Hexan/Essigester 6:1- 5:1) liefert Verbindung 1 a (19.9g, 53%)
   ¹H-NMR (300 MHz, CDCl₃): δ = 1.39, 1.53, 1.66 (3m, 6H, -CH₂-CH₂-CH₂-), 2.45 (s, 3 H, CH₃ₜₒₛ), 3.38 (t, 2H, OCH₂-), 3.93 (m, 2H, O-CH₂-CH = CH₂), 4.01 (t, 2H, OCH₂-), 5.20 (m, 2 H, O-CH₂-CH = CH₂), 5.88 (ₘ, _{1 H}, O-CH₂-CH = CH₂), 7.34, 7.78 (2 m, 4H, Tosyl-Haromat).
b) Synthese von 5-Allyloxy-1-hydroxypentyl-(1→1)-(1R,2R)-trans-1,2-cyclohexandiol (2a): Zu einer Lösung von (1 R,2R)-trans-1,2-Cyclohexandiol (Fluka, 3 g, 25.83 mmol) in DMF (75 ml) gibt man unter Rühren Natriumhydrid (537 mg, 22.4 mmol). Nach 1 h wird Verbindung 1a (4.7 g, 17.22 mmol) in wenig DMF gelöst zugetropft. Nach 18 h wird mit Dichlormethan (500 ml) verdünnt und solange mit Wasser gewaschen bis das Waschwasser neutral reagiert. Die organische Phase wird eingeengt und der Rückstand mittels Flash-Chromatographie (Hexan/Essigester 4:1) gereinigt. Ausbeute: 2.63 g, 63%.
   ¹H-NMR (300 MHz, CDCl₃): δ = 2.82 (bs, 1 H, OH), 3.00 (m, 1 H), 3.96 (m, 2H, O-CH₂-CH = CH₂), 5.22 (m, 2 H, O-CH₂-CH = CH₂), 5.90 (m, 1 H, O-CH₂-CH = CH₂).
c) Synthese von 5-Allyloxy-1-hydroxypentyl-(1→1)-[(2,3,4-tri-O-benzyl-α-L-fucopyranosyl)-(1→2)]-(1R,2R)-trans-1,2-cyclohexandiol (3a):
   Eine Mischung aus 2a (4.6 g, 19 mmol),
   Thioethyl-O-2,3,4-tri-O-benzyl-ß-L-fucopyranosid (13.6 g, 28.51 mmol) und Tetrabutylammoniumbromid (1.4 g, 4.37 mmol) in Dichlormethan (365 ml) und DMF (74 ml) wird 1 h mit Molekularsieb 4 A gerührt. Anschließend wird Kupfer(II)-bromid (7.6 g, 34.2 mmol) zugegeben. Nach 24 h wird über Kieselgur filtriert, mit gesättigter Natriumhydrogencarbonatlösung und danach mit gesättigter Kochsalzlösung gewaschen. Die organische Phase wird über Magnesiumsulfat getrocknet, im Vakuum eingeengt und der Rückstand chromatographiert (Hexan/Essigester 6:1). Ausbeute: 11.9 g (95 %) Verbindung 3a. ¹H-NMR (300 MHz, CDCl₃): δ ₌ 1.10 (d, 3H, 6-H_{fuc}), 4.25 (q, 1H, 5-H_{fuc}), 5.18 (m, 2H, O-CH₂-CH ₌ CH₂), 5.88(m, 1 H, 0-CH2-CH = CH₂).
d) Synthese von 5-Hydroxy-1-hydroxypentyl-(1→1)-[(2,3,4-tri-O-benzyl-α-L-fucopyranosyl)-(1→2)]-(1R,2R)-trans-1,2-cyclohexandiol (4a): Eine Mischung aus Verbindung 3a (15.0 g, 22.78 mmol), Wilkinson-Katalysator (2.1 g, 2.3 mmol) und DBU (2.3 ml) wird in Ethanol/Wasser (9:1, 362 ml) eine Stunde unter Rückfluß gekocht. Danach wird im Vakuum eingeengt und über eine kurze Kieselgelsäule (Hexan/Essigester 4:1) chromatographiert. Der Enolether wird in Aceton/Wasser (9:1, 54 ml) gelöst und mit Quecksilber(II)-oxid (8.3 g) versetzt. Unter Rühren wird Quecksilber(II)-chlorid (8.3 g, 30.75 mmol) gelöst in Aceton/Wasser (9:1, 166 ml) zugetropft. Nach 1 h wird über Kieselgur abgesaugt, mit Chloroform (840 ml) nachgewaschen und mit 30 %iger Kaliumjodidlösung (3 x 170 ml) ausgeschüttelt. Die organische Phase wird über Magnesiumsulfat getrocknet, eingeengt und chromatographiert
   (Hexan/Essigester 2:1→1:1 Ausbeute: (9.53 g, 71 %). ¹H-NMR (300 MHz, CDCl₃): δ ₌ 1.08 (d, 3H, 6-H_{fuc}), 4.24 (q, 1 H, 5-H_{fuc}).
e) Synthese von 5-p-Touosulfonyloxy-1-hydroxypentyl-(1→1)-[(2,3,4-tri-O -benzyl-a-L-fucopyranosyl)-(1→2)]-(1R,2R)-trans-1,2-cyclohexandiol (5a): Zu einer Lösung von Verbindung 4a (2.0 g, 3.23 mmol) in Pyridin (40 ml) gibt man bei 0 C p-
Toluolsulfonsäurechlorid (924 mg, 4.85 mmol). Nach 18 h wird mit Dichlormethan (800 ml) verdünnt, mit gesättigter Natriumchloridlösung (2 x 200 ml) gewaschen und die organische Phase über Natriumsulfat getrocknet und anschließend bei 25°C eingeengt. Flashchromatographie (Hexan/Essigester 5:1 -4:1) liefert Verbindung 5a (1.66 g, 69 %).
   ¹H-NMR (300 MHz, CDCI₃): δ ₌ 1.08 (d, 3H, 6-H_{fuc}), 2.42 (s, 3H, CH_{3tosyl}), 4.19 (q, 1H, 5-H_{fuc}), 4.94 (d, 1 H, 1-H_{fuc}).
f) Synthese von 5-Dimethylmalonyl-1-hydroxypentyl-(1→1)-[(2,3,4-tri-O-benzyl-α-L-fucopyranosyl)-(1→2)]-(1 R,2R)-trans-1,2-cyclohexandiol (6a):
   Eine Mischung aus Verbindung 5a (250 mg, 0.335 mmol), Malonsäuredimethylester (0.3 ml, 2.6 mmol), Kaliumcarbonat (0.18 g, 1.3 mmol) und Dibenzo-18-crown-6 (47 mg, 0.13 mmol) in Toluol (2 ml) wird 24 h bei 100 C gerührt. Das Gemisch wird chromatographiert (Hexan/Essigester 5:1). Man erhält Verbindung 6a (216 mg, 88 %).
   ¹H-NMR (300 MHz, CDCI₃): δ ₌ 1.09 (d, 3H, 6-H_{fuc}), 3.72 (2s, 6H, C(COOMe)₂), 4.22 (q, 1H, 5-H_{fuc}), 4.95 (d, 1 H, 1-H_{fuc}).
g) Synthese von 5-Malonyl-1-hydroxypentyl-(1→1)-[(α-L-fucopyranosy)-(1→2)]-(1R,2R)-trans-1,2-cyclohexandiol (7a):
   Eine Mischung aus Verbindung 6a (180 mg, 0.25 mmol) und Palladiumkohle (10%, 180 mg) in Methanol/Dioxan (10:1, 44 ml) wird 24 h unter Normaldruck in einer Wasserstoffatmosphäre hydriert. Die Palladiumkohle wird abfiltriert, das Filtrat eingeengt und mit 1 M Natronlauge (7 ml) behandelt. Nach 2 h wird mit Amberlite® IR-120 neutralisiert und über Biogel P-2 gereinigt. Man erhält Verbindung 7a (100 mg, 92%).
   ¹H-NMR (300 MHz, D₂₀): δ ₌ 1.07 (d, 3H, 6-H_{fuc}), 1.2 (m, 6H, 4-H_{cyclohex}, 5-H_{c}y_{clohex}, -CH2-), 1.42, 1.55, 1.65, 1.96 (4m, 8H), 4.14 (q, 1 H, 5-H_{fuc}), 4.91 (d, 1 H, 1-H_{fuc}).

### Beispiel 2

a) Synthese von 3-Allyloxy-1-p-toluolsulfonyloxy-propan (1b): Verbindung 1 wird aus Propandiol analog zu Verbindung 1 a synthetisiert.
   ¹H-NMR (300 MHz, CDCl₃): δ = 1.92 (m, 6H, -CH₂-), 2.45 (s, 3 H, CH₃ₜₒₛ), 3.44 (t, 2H, OCH₂-), 3.87 (m, 2H, O-CH₂-CH = CH₂), 4.15 (t, 2H, OCH₂-), 5.20 (m, 2 H, O-CH₂-CH = CH₂), 5.82 (m, 1H, O-CH₂-CH = CH₂), 7.34, 7.79 (2 m, 4H, Tosyl-Hₐᵣₒₘₐₜ).
b) Synthese von 3-Allyloxy-1-hydroxypropyl-(1→1)-(1R,2R)-trans-1,2-cyclohexandiol (2b):
   Verbindung 2b wird analog zu 2a aus 1 b und (1 R,2R)-trans-1,2-cyclohexandiol synthetisiert. ¹H-NMR (300 MHz, CDCl₃): δ = 2.82 (bs, 1H, OH), 3.00 (m, 1H), 3.96 (m, 2H, O-CH₂-CH = CH₂), 5.22 (m, 2 H, O-CH₂-CH = CH₂), 5.90 (m, 1 H, O-CH₂-CH = CH₂).
c) Synthese von 3-Allyloxy-1-hydroxypropyl-(1→1)-[(2,3,4-tri-O-benzyl-α-L-fucopyranosyl)-(1→2)]-(1R,2R)-trans-1,2-cyclohexandiol (3b):
   Verbindung 3b wird analog zu Verbindung 3a aus 2b und Thioethyl-O-2,3,4-tri-O-benzyl-ß-L-fucopyrano- sid synthetisiert.
   ¹H-NMR (300 MHz, CDCl₃): δ ₌ 1.10 (d, 3H, 6-H_{fuc}), 4.23 (q, 1H, 5-H_{fuc}), 5.18 (m, 2H, O-CH₂-CH ₌ CH₂), 5.86(m, 1H, O-CH₂-CH = CH₂).
d) Synthese von 3-Hydroxy-1-hydroxypropyl-(1→1)-[(2,3,4-tri-O-benzyl-α-L-fucopyranosyl)-(1→2)]-(1 R,2R)-trans-1,2-cyclohexandiol (4b):
   Verbindung 4b wird durch Deallylierung von 3b entsprechend der Synthese von 4a synthetisiert.
   ¹H-NMR (300 MHz, CDCl₃): δ ₌ 1.09 (d, 3H, 6-H_{fuc}), 4.23 (^{q}, 1H, 5-H_{fuc}).
e) Synthese von 3-p-Toluolsulfonyloxy-1-hydroxypropyl-(1→1)-[(2,3,4-tri-O -benzyl-a-L-fucopyranosyl)-(1→2)]-(1R,2R)-trans-1,2-cyclohexandiol (5b):
   Die Tosylierung von Verbindung 4b wird analog zur Synthese von 5a durchgeführt.
   ¹H-NMR (300 MHz, CDCl₃): δ ₌ 1.05 (d, 3H, 6-H_{fuc}), 2.41 (s, 3H, CH_{3tosyl}), 4.94 (d, 1H, 1-H_{fuc}). f) Synthese von 3-Dimethylmalonyl-1-hydroxypropyl-(1→1)-[(2,3,4-tri-O-benzyl-α-L-fucopyranosyl)-(1→2)]-
(1 R,2R)-trans-1,2-cyclohexandiol (6b):
   Verbindung 6b wird analog zu Verbindung 6a synthetisiert.
   ¹H-NMR (300 MHz, CDCl₃): δ ₌ 1.11 (d, 3H, 6-H_{fuc}), 3.69 (s, 6H, C(COOMe)₂), 4.21 (q, 1H, 5-H_{fuc}), 4.96
   (d, 1 H, 1-H_{fuc}).

   g) Synthese von 3-Malonyl-1-hydroxypropyl-(1→1)-[(α-L-fucopyranosyl)-(1→2)]-(1R,2R)-trans-1,2-cyclohexandiol (7b):
   Verbindung 6b wird analog zu 6a entschützt.
   ¹H-NMR (300 MHz, D₂₀): δ = 1.06 (d, 3H, 6-H_{fuc}), 1.06 (ₘ, 4H, 4-H_{c}y_{clohex}, 5-H_{c}y_{clohex}), 1.47, 1.56, 1.76, 1.98, (4m, 8H), 4.13 (q, 1H, 5-H_{fuc}), 4.90 (d, 1H, 1-H_{f}uc).
   Beispiel 3 Synthese von 3-Malonyl-1-hydroxybutyl-(1→1)-[(α-L-fucopyranosyl)-(1→2)]-(1R,2R)-trans-1,2-cyclohexandiol (7c):
      Verbindung 7c wird analog zu den Verbindungen 7a und 7b hergestellt.

### Beispiel 4

a) Synthese von [(2,3,4,-tri-O-benzyl-a-L-fucopyranosyl)-(1 1)]-(1R,2R)-trans-1,2-cyclohexandiol (1 d):
Eine Mischung aus (1 R,2R)-trans-1,2-cyclohexandiol (2,43 g, 20,9 mmol), Thioethyl- O-2,3,4-tri-O-benzyl-β-L-fucopyranosid (8,0 g, 16,72 mmol) und Tetrabutylammoniumbromid (2,7 g, 8,36 mmol) in Dichlormethan (200 ml) und DMF (40 ml) wird 1 mit Molekularsieb 4 A gerührt. Anschließend wird Kupfer(II)-bromid (5,6 g, 25,08 mmol) zugegeben. Nach 24 h wird wie unter 3a beschrieben aufgearbeitet und mit Hexan/Essigester 3:1 chromatographiert. Ausbeute (6,8 g, 76%)
   ¹H-NMR (300 MHz, CDCl₃): δ ₌ 1.13 (d, 3H, 6-H_{fuc}), 1.21 (m, 4H, 4-H_{cyclohex}, 5-H_{c}y_{clohex}), 1.65, 2.01 (2m, 4H, 3-H_{c}y_{cl}oₕex, 6-^{H}cyclohex )
b) Synthese von 1-Hydroxy-4-aminocarbonylbutyl-(4 2)-[(2,3,4,-tri-O-benzyl-α-L-fucopyranosyl)-( 1 1)]-(1 R,2R)-trans-1,2-cyclohexandiol (2d):
   Zu einer Lösung aus 1 c (1,5 g, 2,8 mmol) in Dichlormethan (30 ml), gibt man Triethylamin (854 µl, 3,08 mmol), DMAP (38 mg, 0,308 mmol) und Chlorameisensäurenitrophenylester (1,128 g, 2,8 mmol). Die Mischung wird über Nacht gerührt und mit N-Ethyldiisopropylamin (599 µl, 3,5 mmol) sowie 4-Amino-1-butanol (325 µl, 3,5 mmol) versetzt. Es wird nochmal 18 h gerührt. Zur Aufarbeitung wird mit Dichlormethan (70 ml) verdünnt und mit Wasser (3 x 30 ml) gewaschen. Die organische Phase wird im Vakuum eingeengt und mit Toluol/Aceton 3:1 chromatographiert. Ausbeute (1,4 g, 77%).
   ¹H-NMR (300 MHz, CDCl₃): δ ₌ 1.08 (d, 3H, 6-H_{fuc}), 1.30 (m, 4H, 4-H_{c}y_{clohex}, 5-H_{cyclohex}), 1.53 (m, 4H,
   -CH₂-CH₂-), 1.65, 2.01 (2m, 4H, 3-H_{c}y_{cloh}ex, 6-H_{cyclohex} ),
c) Synthese von 4-Malonyl-1-aminocarbonylbutyl-(4 2)-[(a-L-fucopyranosyl)-(1 1)]-(1R,2R)-trans-1,2-cyclohexandiol (3d):
   Verbindung 3d wird analog zu Verbindung 7a hergestellt (4 Stufen aus 2d: tosylieren, malonylieren, hydrieren, verseifen), mit dem Unterschied, daß die Umsetzung des aus 2d hergestellten Tosylats mit Malonsäuredimethylester bei 60 °C (5 h) durchgeführt wird. Die Hydrogenolyse der Benzylgruppen verläuft völlig analog, die Verseifung des Malonesters erfolgt in MeOH/1 M wässriger NaOH 2:3 (2 h). ¹H-NMR (300 MHz, D₂0): δ ₌ 1.08 (d, 3H, 6-H_{fuc}), 1.30 (m, 4H, 4-H_{c}y_{clohex}, 5-H_{cyclohex}), 1.53 (m, 4H, -^{CH}2-^{CH}2-), ^{1.65, 2.01} (²m, ^{4H,} 3-H_{c}y_{clohex}, 6-H_{cyclohex} ), 1.13 (m, 4H), 1,37 (m,4H), 1,60 (m, 4 H), 1.80, ₂._{01 (2m}, _{2H)}, _{3.90 (}q, 1H, ₅-_{Hfuc}), ₄.₄₉ (m, 1H, 2-H_{cyclohex}), 4.89 (bs, 1H, 1-H_{f}uc)

### Beispiel 5

### Synthese von (1 R,2R)-trans-1,2-Cyclohexandiol-O-2,3,4,6-tetra-O-acetyl-β-D-galactopyranosid (1 e)

Zu einer Lösung von O-(2,3,4,6-Tetra-O-acetyl-D-galactopyranosyl)-trichloracetimidat (11.29 g, 22.9 mmol) und Cyclohexandiol (4.0 g, 34.35 mmol) in Dichlormethan/Ether (100:200 ml) gibt man eine 1 M Trimethylsilyltrifluormethansulfonat-Lösung (2.3 ml). Nach 1 h wird mit Natriumhydrogencarbonat (1 g) neutralisiert, filtriert und im Vakuum eingeengt. Chromatographie des Rückstandes (Toluol/Aceton 5 :1) liefert 1e (7.4g, 72%).

Synthese von (1 R,2R)-trans-1,2-Cyclohexandiol-O-(2,3,4-tri-O-benzyl-α-L-fucopyranosyl)-2,3,4,6-tetra-O-acetyl-ß-D-galactopyranosid (2e)

Verbindung 2e wird analog zu 3a synthetisiert.

Synthese von (1 R,2R)-trans-1,2-Cyclohexandiol-O-(2,3,4-tri-O-benzyl-α-L-fucopyranosyl)-β-D-galactopyrano- sid (3e)

Eine Lösung von 2e (7.6 g, 8.8 mmol) in Methanol (360 ml) wird mit einer 2 M methanolischen Natriummethanolatlösung (1.4 ml) behandelt. Nach 3 h wird mit Amberlite IR-120 neutralisiert, filtriert, eingeengt und der Rückstand chromatographiert (Dichlormethan/Methanol 25:1«20:1). Man erhält 3e (6.0 g, 98%).

Synthese von (1 R,2R)-trans-1,2-Cyclohexandiol-O-(2,3,4-tri-O-benzyl-α-L-fucopyranosyl)-β-D-3-O-benzyl-ga- lactopyranosid (4e)

Eine Lösung von 3e (2 g, 2.88 mmol) und Dibutylzinnoxyd (0.86 g, 3.45 mmol) in Methanol (31 ml) wird unter Rückfluß gekocht. Nach 18 h wird eingengt und mit Toluol coevaporiert. Der Rückstand wird in Toluol (38 ml) gelöst, mit Benzylbromid (3.5 ml, 29.9 mmol) und Tetrabutylammoniumiodid (1.34 g, 3.63 mmol) versetzt und auf 45 °C erwärmt. Nach 3h wird eingeengt und der Rückstand mit Dichlormethan/Methanol 25:1 chromatographiert.

Ausbeute: 1.6 g (71%).

¹H-NMR (300 MHz, CDCl₃): δ ₌ 1.09 (d, 3H, 6-H_{fuc}), 2.48 (d, 1 H, OH), 2.55 (bs, 1 H, OH), 4.30 (d, 1 H, 1-Hgal).

Synthese von (1R,2R)-trans-1,2-Cyclohexandiol-O-(2,3,4-tri-O-benzyl-α-L-fucopyranosyl)-β-D-3-O-benzyl-4,6-O-benzyliden-galactopyranosid (5e)

Zu einer Lösung aus 4e ( 1.16 g, 1.47 mmol) in Acetonitril (63 ml) gibt man Benzaldehyddimethylacetal ( 0.44 ml, 2.94 mmol) und p-Toluolsulfonsäure (84 mg). Nach 1 h bricht man die Reaktion mit Kaliumcarbonat (1 g) ab, filtriert, engt ein und chromatographiert den Rückstand mit Hexan/Essigester 3:1→2:1→1:1. Man erhält 5e (1.14 g, 89%).

1 H-NMR (300 MHz, CDCI3): δ = 1.03 (d, 3H, 6-Hfuc), 2.39 (d, 1 H, OH), 5.50 (s, 1 H, CHPh).

Synthese von (1 R,2R)-trans-1,2-Cyclohexandiol-O-(α-L-fucopyranosyl)-β-D-2-O-malonylethyl-galactopyrano- sid (6e)

Verbindung 6e wird wie bei 7a beschrieben synthetisiert (Alkylierung mit 2-Allyloxy-1-p-toluolsulfony- loxy-ethan, Deallylierung, Tosylierung, Malonylierung, Hydrogenolyse, Verseifung).

¹H-NMR (300 MHz, D₂0): δ ₌ 1.04 (d, 3H, 6-H_{fuc}), 4.38 (d, 1 H, 1-Hgₐₗ), 4.45 (q, 1 H, 5-H_{fuc}), 4.86 (d, 1H, 1-H_{fuc}).

### Beispiel 6

Synthese von 4,6-Isopropyliden-1,2-didesoxyglucose (1f):
Eine Lösung von Tri-O-acetyl-D-glucal (30 g, 110.17 mmol) in Dioxan (400 ml) wird mit Palladiumkohle (10%, 3g) 24 h in einer Wasserstoffatmosphäre hydriert. Die Mischung wird durch Kieselgur filtriert und eingeengt. Zur Abspaltung der Acetylgruppen wird der Rückstand in Methanol (500 ml) aufgenommen und eine 1 M Natriummethanolatlösung (6 ml) zugegeben. Nach 90 min wird mit Amberlite IR-120 neutralisiert, filtriert und im Vakuum eingeengt. Der Rückstand wird mit Toluol (3 x 250 ml) coevaporiert und in DMF (500 ml) aufgenommen. Zu der Lösung gibt man Dimethoxypropan (140 ml, 114,6 mmol) und p-Toluolsulfonsäure (400 mg). Nach 18 h gibt man Triethylamin (3 ml) zu, läßt noch 15 min rühren und engt im Hochvakuum ein. Chromatographie (Toluol/Aceton 4:1) liefert 1f (33 g, 80 %).
¹H-NMR (300 MHz, CDCl₃): δ = 1.41, 1.51 (2s, 6H, 2 CH₃), 1.76 (ddd, 1H, 2-H), 2.0 (ddd, 1H, 2-H), 2.8 (d, 1 H, OH), 3.16 (m, 1 H, 1-H), 3.46 (dd, 1 H, 6-H), 3.53 (m, 1 H, 1-H), 3.7 (dd, 1 H, 6-H), 3.86 (dd, 1 H, 4-H), 3,96 (m, 1 H, 5-H).

Synthese von

4-Allyloxy-1-hydroxybutyl-(1 3)-4,6-Isopropyliden-1,2-didesoxyglucose (2f):

2f wird analog zu 2a synthetisiert.

Synthese von 4-Allyloxy-1-hydroxybutyl-(1 3)-1,2-didesoxyglucose (3f): Zu einer Lösung aus 2a (4.17 g, 14 mmol) in Dichlormethan (340 ml) gibt man 20%ige Trifluoressigsäure (30 ml). Nach 4 h gibt man Toluol (200 ml) dazu und engt auf die Hälfte ein. Man gibt erneut Toluol dazu und engt ein. Der Rückstand wird mit Dichlormethan/Methanol (50:1 40:1 30:1) chromatographiert. Man erhält 3f (3.26 g, 90%).

¹H-NMR (300 MHz, CDCl₃): δ = 1.53 (ddd, 1H, 2-H), 1.66 (m, 4H, CH₂CH₂) 2.0 (ddd, 1H, 2-H), 2.9 (bs, 1H, OH), 5.22 (m, 2H, O-CH₂-CH = CH₂), 5.9 (m, 1 H, O-CH₂-CH = CH₂).

Synthese von 4-Phenyl-1-trifluormethansulfonyloxy-butan (4f)

Zu einer eiskalten Lösung von Trifluormethansulfonsäureanhydrid (3.8 ml, 23 mmol) in Dichlormethan (35 ml) tropft man unter Rühren eine Mischung aus 4-Phenyl-1-butanol (3ml, 20 mmol), Pyridin (1.6 ml, 20 mmol) und Dichlormethan (10 ml). Nach 1 h gibt man Dichlormethan (65 ml) zu und wäscht mit Wasser (3 x 20 ml), trocknet über Magnesiumsulfat und engt bei 25 °C im Vakuum ein. Der Rückstand wird mit Hexan/Essigester 7:1 chromatographiert. Man erhält 4f (3.8 g, 70 %).

¹H-NMR (300 MHz, CDCl₃): δ = 1.84 (m, 4 H, -CH₂-CH₂-), 2.67 (t, 2 H, -CH₂-Ph), 4.52 ( t, 2H, -CH₂-OTf), 7.24 (m, 5H, Ph).

Synthese von 4-Allyloxy-1-hydroxybutyl-(1→3)-[4-phenyl-1hydroxybutyl-(1→6)]-1,2-didesoxyglucose (5f):
Eine Mischung aus 3f (850 mg, 3.3 mmol), 4f (1.21 g, 4.3 mmol), Kaliumcarbonat (684 mg, 4.95 mmol) und Dibenzo-18-crown-6 (174 mg, 480 µmol) wird 18 h in Toluol (14 ml) gerührt. Zur Aufarbeitung wird filtriert und chromatographiert (Toluol/Aceton 10:1). Ausbeute: 942 mg (73%).
¹H-NMR (300 MHz, CDCl₃): δ = 1.53 (ddd, 1H, 2-H), 1.66 (m, 8 H, 4 CH₂), 2.00 (m, 1 H, 2-H), 2.60 (dd, 2H, CH₂Ph), 2.87 (bs, 1 H, OH), 3.95 (m, 2H, O-CH₂-CH = CH₂), 5.22 (m, 2H, O-CH₂-CH = CH₂), 5.91 (m, 1H, O-CH₂-CH = CH₂).

Synthese von 4-Allyloxy-1-hydroxybutyl-(1 3)-[(2,3,4,-tri-O-benzyl-a-L-fucopyranosyl)-(1 4)]-[ 4-phenyl-1-hydroxybutyl-(1 6)]-1,2-didesoxyglucose (6f):
Die Fucosylierung erfolgt wie bei 3a beschrieben.

Synthese von 4-Malonyl-1-hydroxybutyl-(1 3)-[(a-L-fucopyranosyl)-(1 4)]-[4-phenyl-1-hydroxybutyl-(1 6)]-1,2- didesoxyglucose (7f):
7f wird analog zu 7a synthetisiert (5 Stufen aus 6f: deallylieren, tosylieren, malonylieren, hydrieren, verseifen).

1H-NMR (300 MHz, D₂0): δ = 1.0 (d, 3H, 6-H_{fuc}), 2.50 (t, 2H, CH₂ Ph), 2.90 (t, 2H), 4.20 (q, 1 H, 5-H_{fuc}), 4.70 (d, 1 H, 1-H_{fuc}).

## Patentansprüche

1. Verbindung der Formel I 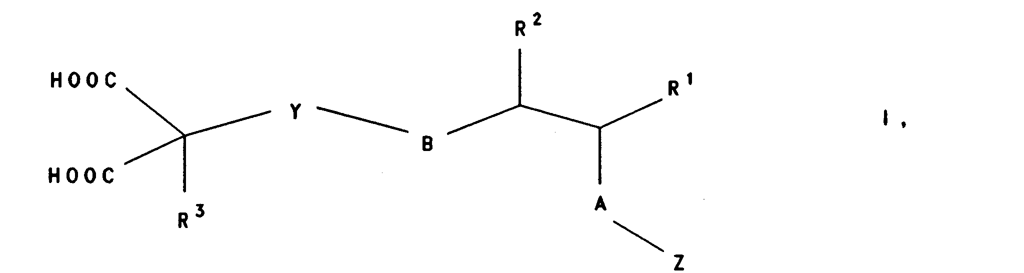 in der
R¹, R² und R³ unabhängig voneinander H, (CH₂)ₘX oder CH₂O(CH₂)ₘX¹ bedeuten oder R¹ und R² gemeinsam einen sechsgliedrigen Carbo- oder Heterocyclus mit mindestens einem der Substituenten R⁴, R⁵ und R⁶ bilden und
A und B unabhängig voneinander O, S, NH, HN-CO, OC-NH, O-CO, OC-O, NH-CO-O, O-CO-NH, S-CO, SC-0, O-CS-S, S-CS-0, NH-CS-S, S-CS-NH oder CH₂ bedeuten und
Z eine Pyranose, eine Furanose, einen offenkettigen Polyalkohol darstellt oder Y-X⁶ bedeutet,
Y -O-(CX²,X³)ₙ, -(CX²,X³)ₙ, -(CX²,R⁷)ₙ-, -(CR⁷,R⁸)ₙ-, - CH₂-(CX²,X³)⁻ₙ oder einen gesättigten oder ungesättigten, sechsgliedrigen Carbo- oder Heterozyklus mit mindestens einem Substituenten R⁹ oder eine Kombination aus der Kette -0-(CX²,X³)ₙ,-(CX²,X³)ₙ, -(CX²,R⁷)ₙ-, -(CR⁷,R⁸)ₙ- und dem Carbo- oder Heterozyklus darstellt, wobei
R⁴, R⁵ und R⁶ unabhängig voneinander H, OH, (CH₂)qX⁴,CH₂O(CH₂)_{q}X⁵ oder HNC(O)-CH₃ und
R⁷, R⁸ und R⁹ unabhängig voneinander H, OH, (CH₂)qX⁴ oder CH₂0(CH₂)qX⁵ und
X, X¹, X², X³, X⁴ und X⁵ unabhängig voneinander H, NH₂, COOH, OH, CH₂0H, CH₂NH₂, C₁-C₂₀-Alkyl oder C₆-C₁₀-Aryl und
_{X}6 OH oder 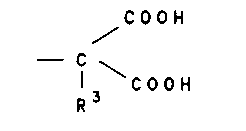
und
m, n und q unabhängig voneinander eine Zahl von 1 bis 20 bedeuten.

2. Verbindung nach Anspruch 1, dadurch gekennzeichnet, daß
R¹ und R² einen sechsgliedrigen Carbocyclus bilden und
R³, R⁴, R⁵ und R⁶ H,
A O,
Z eine -Fucopyranosyl-Gruppe,
Y (CX²,X³)ₙ und
X² und X³ H bedeuten.

3. Verbindung nach Anspruch 2, dadurch gekennzeichnet, daß B O bedeutet.

4. Verbindung nach Anspruch 3, dadurch gekennzeichnet, daß n für 3 steht.

5. Verbindung nach Anspruch 3, dadurch gekennzeichnet, daß n für 4 steht.

6. Verbindung nach Anspruch 3, dadurch gekennzeichnet, daß n für 5 steht.

7. Verbindung nach Anspruch 2, dadurch gekennzeichnet, daß B HNCO-O bedeutet.

8. Verbindung nach Anspruch 7, dadurch gekennzeichnet, daß n für 4 steht.

9. Verbindung nach Anspruch 1, dadurch gekennzeichnet, daß
R¹ und R² einen sechsgliedrigen Carbocyclus bilden und
R³, R⁴, R⁵ und R⁶ H,
A und B O,
Z eine Fucopyranosyl-Gruppe,
Y eine Kombination aus einer Kette -O-(CX²,X³)ₙ und einem Heterozyklus darstellt, wobei
X² und X³ H bedeuten.

10. Verbindung nach Anspruch 9, dadurch gekennzeichnet, daß der Heterocyclus ein Galactosylrest ist und n für 2 steht.

11. Verbindung nach Anspruch 1, dadurch gekennzeichnet, daß R₁ und R₂ gemeinsam einen substituierten
Tetrahydropyranring bilden,
A und B O,
Z eine -Fucopyranosyl-Gruppe, Y (CX²,X³)ₙ bedeuten, wobei
R³, X² und X³ H bedeuten und n für 4 steht.

12. Verbindung nach Anspruch 11, dadurch gekennzeichnet, daß die Substituenten des Tetrahydropyranrings
R⁴ und R⁵ H und
der im Tetrahydropyranring zwischen dem Ringheteroatom O und dem Ringsubstituenten -A-Z liegende Substituent
R6 CH₂O(CH₂)qX⁵ und
X⁵ C₆ H₅ bedeuten.

13. Verbindung nach Anspruch 12, dadurch gekennzeichnet, daß q für 4 steht.

14. Verbindung nach Anspruch 11, dadurch gekennzeichnet, daß der Tetrahydropyranring eine Pyranose ist.

15. Verbindung nach Anspruch 12, dadurch gekennzeichnet, daß die Pyranose N-Acetyl-D-glucosamin ist.

16. Verbindung der Formel 1 nach Anspruch 1, dadurch gekennzeichnet, daß R¹ und R² gemeinsam einen sechsgliedrigen Carbocyclus bilden,
A-Z 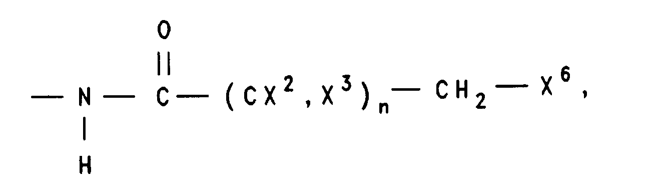
-B-Y- 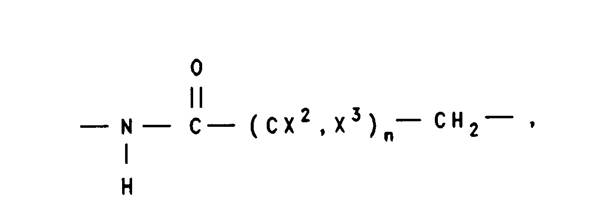
R³ und X² H,
X³ und X⁶ OH und
n 4 bedeuten.

17. Verbindung nach Anspruch 16, dadurch gekennzeichnet, daß X6 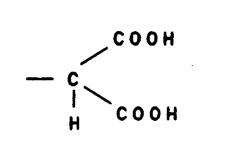 bedeutet.

18. Verfahren zur Herstellung einer Verbindung der Formel I gemäß eines der Ansprüche 1 bis 17, dadurch gekennzeichnet, daß man zunächst unter Alkylierung, Acylierung oder Glycosylierung einer funktionellen Gruppe eines Akzeptors II, 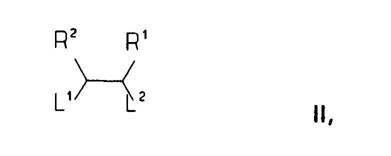 aufweisend mindestens zwei benachbarte funktionelle Gruppen L¹ und L² sowie aufweisend die Substituenten R¹ und R², mittels eines Äquivalentes eines mindestens zwei funktionelle Gruppen L³ und L⁴ tragenden Donors III, 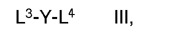 dessen eine funktionelle Gruppe L³ notwendigenfalls geschützt und dessen andere funktionelle Gruppe L⁴ gebenenfalls aktiviert vorliegt, Zwischenverbindung IV, 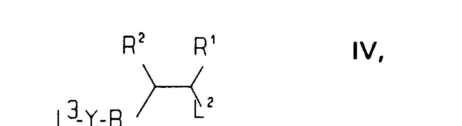 herstellt, wonach man unter Alkylierung, Acylierung oder Glycosylierung der ungeschützten funktionellen Gruppe L² der Zwischenverbindung IV mittels eines mit einer aktivierten funktionellen Gruppe L⁵ versehenen Donors V, 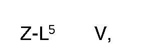 dessen übrige funktionelle Gruppen notwendigenfalls Schutzgruppen tragen, Zwischenverbindung VI, 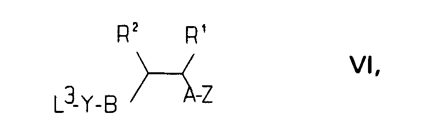 herstellt und schließlich gegebenenfalls nach selektiver Entschützung und Aktivierung der funktionellen Gruppe L³ durch Umsetzung der Zwischenverbindung VI mit einem Malonsäurederivat und nachfolgender Abspaltung sämtlicher Schutzgruppen die Verbindung der Formel I gemäß Anspruch 1 erhält, wobei die Variablen R¹, R², Y, B, Z und A die in Anspruch 1 genannte Bedeutung haben.

19. Verfahren nach Anspruch 18, dadurch gekennzeichnet, daß man Akzeptor II zunächst mit Donor V und anschließend mit Donor III zur Zwischenverbindung VI umsetzt.

20. Verfahren nach Anspruch 18 oder 19 zur Herstellung einer Verbindung gemäß Anspruch 3, dadurch gekennzeichnet, daß
Akzeptor II (1 R,2R)-trans-1,2-Cyclohexandiol,
Donor II ein n-Allyloxy-1-p-toluolsulfonyloxy-(C₂-Cₙ)-alkan und
Donor V Thioethyl-O-2,3,4-tri-O-benzyl-ß-L-fucopyranosid ist.

21. Verfahren nach Anspruch 19 zur Herstellung einer Verbindung gemäß Anspruch 7, dadurch gekennzeichnet, daß
Akzeptor II (1 R,2R)-trans-1,2-Cyclohexandiol,
Donor II ein mit Chlorameisensäure-p-nitrophenylester aktivertes Aminoalkanol und
Donor V Thioethyl-O-2,3,4-tri-O-benzyl-ß-L-fucopyranosid ist.

22. Verfahren nach Anspruch 18 oder 19 zur Herstellung einer Verbindung gemäß Anspruch 16, dadurch gekennzeichnet, daß
Akzeptor II (1 R,2R)-trans-1,2-Cyclohexandiamin und
Donor III und IV Glucono-1,5-lacton ist.

23. Verfahren nach Anspruch 18 oder 19 zur Herstellung einer Verbindung gemäß Anspruch 17, dadurch gekennzeichnet, daß
Akzeptor II (1 R,2R)-trans-1,2-Cyclohexandiamin,
Donor III und V Glucono-1,5-lacton ist und
daß nach selektiver Entschützung und Aktivierung der endständigen funktionellen Gruppen jeweils an Y und an Z Zwischenverbindung VI mit zwei Äquivalenten eines entsprechenden Malonsäurederivates umgesetzt wird.

24. Arzneimittel, enthaltend eine Verbindung gemäß eines der Ansprüche 1 bis 17 und gegebenfalls pharmazeutische Träger.

25. Verwendung einer Verbindung der Formel I gemäß eines der Ansprüche 1 bis 17 zur Herstellung eines Arzneimittels zur Therapie oder Prophylaxe von Krankheiten, bei welchen bakterielle oder virale Infektionen, entzündliche Prozesse oder metastasierende Tumoren involviert sind.

26. Verwendung einer Verbindung der Formel I gemäß eines der Ansprüche 1 bis 17 zur Herstellung eines Mittels zur Diagnose von Krankheiten, bei welchen bakterielle oder virale Infektionen, entzündliche Prozesse oder metastasierende Tumoren involviert sind.
